# EUROPEAN PATENT SPECIFICATION

(11) **EP 1 711 158 B1**
(45) Date of publication and mention of the grant of the patent: **27.05.2009**
(21) Application number: 04806793.8
(22) Date of filing: 16.11.2004
(51) Int. Cl.: A61K 9/00, A61P 31/00, A61K 31/155, A61K 31/198

(54) **OTIC DETERGENT COMPOSITION FOR ANIMALS, WITH EDTA AND CHLOREXHIDINE**
OHR-SPÜLZUSAMMENSETZUNG FÜR TIERE MIT EDTA UND CHLORHEXIDIN
COMPOSITION DETERGENTE OTIQUE POUR ANIMAUX COMPORTANT DE L'EDTA ET DE LA CHLOREXHIDINE

(30) Priority: 05.02.2004 IT CR20040004
(43) Date of publication of application: 18.10.2006
(73) Proprietor: I.C.F. Industria Chimica Fine S.r.l., 26020 Palazzo Pignano (IT)
(72) Inventor: FALANGA, Gennaro, I-26040 Gerre De'Caprioli (IT)
(74) Representative: Marcio', Paola
(86) International application number: PCT/IT2004/000627
(87) International publication number: WO 2005/074886

(56) References cited:
- DE-A1- 3 007 397
- US-A- 4 354 952
- US-A1- 2002 177 577
- US-B1- 6 284 749
- US-B1- 6 365 131
- US-B1- 6 482 799
- MERYON S D ET AL: "SMEAR REMOVAL AGENTS: A QUANTITATIVE STUDY IN VIVO AND IN VITRO" JOURNAL OF PROSTHETIC DENTISTRY, XX, XX, vol. 57, no. 2, 1 February 1987 (1987-02-01), pages 174-179, XP000646397 ISSN: 0022-3913
- NIELLOUD, F. ET AL: "Development of an in vitro test to evaluate cerumen dissolving properties of several veterinary ear cleansing solutions" JOURNAL OF DRUG DELIVERY SCIENCE AND TECHNOLOGY , 14(3), 235-238 CODEN: JDDSAL, 2004, XP008043830

## Description

The present invention relates to a new otic detergent composition useful for animals, in particular for dogs and cats. The field of the invention is that of otic detergent compositions for the treatment of external otitis of animals such as dogs and cats.

Pharmaceutical products intended for topical administration to eyes, nose or ears, are known e.g. from US 6,284,749.

Known products for this purpose generally make also use of chlorhexidine as active principle, but they are effective only at high concentration of chlorhexidine, giving undesired collateral effects.

The aim of the invention is to provide a new otic detergent composition for animals, in particular for dogs and cats, to be effective at a lower concentration of the active principle chlorhexidine, thus being more tolerable.

This is achieved by an otic detergent composition according to claim 1. Preferred compositions are further disclosed by the dependent claims.

Compared to known otic detergents for dogs and cats, the claimed composition is effective at a lower concentration of the active principle, thanks to the synergism between chlorhexidine and the tris(hydroxymethyl) aminomethane -EDTA-derived buffer; undesired collateral effects are thus reduced. The claimed composition is useful for cleaning the auditory canal of animals like dogs and cats, for removing bacteria sensitive to chlorhexidine, and for preventing otic pathologies or diseases.

The active principle according to the invention is a chlorhexidine-derived compound, which is chlorhexidine digluconate C₃₄H₅₄C₁₂N₁₀O₁₄, or chlorhexidine dichlorhydrate C₂₂H₃₈C₁₄N₁₀ or chlorhexidine diacetate C₂₆H₃₈ₐC₁₂N₁₀O₂ , in solution.

According to the invention, the composition comprises a tris(hydroxymethyl) aminomethane - EDTA-derived buffer, the EDTA-derived compound is EDTA disodium salt dihydrate Na₂H₂EDT·A2H₂O (disodium EDTA) or ethylenediaminetetraacetic acid H₄EDTA.

The composition further comprises an acidifying or alcalizing agent to reach pH 8, and a wetting agent. An acidifying or alcalizing agent is used, respectively, when the aforesaid buffer is obtained with disodium EDTA or with the acid H₄EDTA; the preferred acidifying agent is lactic acid; the preferred wetting agent is propylene glycol C₃H₈O₂.

According to a preferred composition, 100 g comprise:
- 1 to 20 g propylene glycol;
- 0,00001 to 0,8 g chlorhexidine digluconate at 20% solution;
- 0,001 to 0,12 g disodium EDTA;
- 0,001 to 0,60 g tris(hydroxymethyl) aminomethane;
- lactic acid at 80% solution, to reach pH 8;
- demineralized water q.s. to 100 g.

Advantageously, low or very low concentrations of chlorhexidine digluconate correspond to concentrations of disodium EDTA and tris(hydroxymethyl) aminomethane close to above maximum values, and vice versa.

According to a more preferred composition, 100 g comprise:
- 5 to 15 g propylene glycol;
- 0,1 to 0,8 g chlorhexidine digluconate at 20% solution;
- 0,001 to 0,12 g disodium EDTA;
- 0,001 to 0,60 g tris(hydroxymethyl) aminomethane;
- lactic acid at 80% solution, to reach pH 8;
- demineralized water q.s. to 100 g.

According to an even more preferred composition, 100 g comprise:
- 10 g propylene glycol;
- 0,75 g chlorhexidine digluconate at 20% solution;
- 0,001 g disodium EDTA;
- 0,0038 g tris(hydroxymethyl) aminomethane;
- lactic acid at 80% solution, to reach pH 8;
- demineralized water q.s. to 100 g.

The use is as follows: the detergent composition is poured into the auditory canal; then the ear is massaged to favour the exit of the detergent mixed with earwax; the auditory canal is then carefully cleaned and dried with cotton or with a gauze.

Results from in vivo test of the otic detergent composition according to the present invention are given hereinbelow. The purpose was to test the tolerability and antimicrobial activity of the composition of the invention, comprising chlorhexidine digluconate (0,15% solution), in the presence of the tris(hydroxymethyl) aminomethane - disodium EDTA buffer (tris-EDTA buffer), for treatment of chronic, bacterial external otitis of dogs.

A lot of 11 dogs was treated with a solution of the detergent composition according to the invention, once a day for a period of two weeks. Dogs were evaluated at initial conditions (G0), after 14 days (G14) and after 28 days (G28).

Three clinical parameters (exudates, erythema, pain) were evaluated, together with three cytologic parameters (*Malassezia*, cocci, bacilli) by otoscopic and citologic test of the exudate. Bacterial cultures were prepared for each test, and in the cases when bacteria were still found at the citologic test after 14 days, dogs were treated with the detergent composition of the invention and 10 minutes after with Enrofloxacin 5% solution, for two weeks. At G0 11/11 cases revealed gram-negative resistant bacteria, and 6/11 cases revealed gram-positive bacteria.

At G14, 6 of 11 tests of the cultures were negative; at G28 10 of 11 tests were negative, and only one revealed a positive culture. At G14, clinical and microbiological (bacterial) values were reduced by 54,6% and 71,1% respectively; at G28 they were reduced by 85,7% and 94%. Good tolerance was observed in 100% cases.

These results showed that the otic detergent composition helps keeping under control the chronic, bacterial external otitis of dogs. Tests also showed excellent tolerability of the product, and confirmed very good results in the combination tris-EDTA / chlorhexidine digluconate 0,15% with the antimicrobial agent (Enrofloxacin) against gram-positive and gram-negative resistant bacteria.

## Claims

1. Otic detergent composition for animals and in particular for dogs and cats, comprising a chlorhexidine-based active agent, and a tris(hydroxymethyl)aminomethane - EDTA-derivative buffer, said chlorhexidine-based active agent being a solution of chlorhexidine digluconate C₃₄H₅₄C₁₂N₁₀O₁₄, or chlorhexidine dichlorhydrate C₂₂H₃₈C₁₄N₁₀, or chlorhexidine diacetate C₂₆H₃₈C₁₂N₁₀O₂, said EDTA-derived buffer being dihydrate disodium EDTA Na₂H₂EDTA.2H₂O or ethylenediaminetetracetic acid H₄EDTA.

2. Composition according to claim 1, further comprising an acidifying or alcalizing agent so that pH of said composition is equal to 8.

3. Composition according to claim 2, wherein said acidifying agent is lactic acid.

4. Composition according to any one of claims 1 to 3, wherein 100 g of said composition comprise:
- 1 to 20 g propylene glycol;
- 0,00001 to 0,8 g chlorhexidine digluconate at 20% solution;
- 0,001 to 0,12 g disodium EDTA;
- 0,001 to 0,60 g tris(hydroxymethyl) aminomethane;
- lactic acid at 80% solution, to reach pH 8;
- demineralized water q.s. to 100 g.

5. Composition according to claim 4, wherein 100 g of said composition comprise:
- 5 to15 g propylene glycol;
- 0,1-0,8 g chlorhexidine digluconate at 20% solution;
- 0,001-0,12 g disodium EDTA;
- 0,001-0,60g tris(hydroxymethyl) aminomethane;
- lactic acid at 80% solution, to reach pH 8;
- demineralized water q.s. to 100g

6. Composition according to claim 4, wherein 100 g of said composition comprise
- 10 g propylene glycol;
- 0,75 g chlorhexidine digluconate at 20% solution;
- 0,001 g disodium EDTA;
- 0,0038 g tris(hydroxymethyl) aminomethane;
- lactic acid at 80% solution, to reach pH 8;
- demineralized water q.s. to 100 g.

## Patentansprüche

1. Ohrenreinigungsmittel für Tiere, insbesondere für Hunde und Katzen, mit einem Wirkstoff auf der Basis von Chlorhexidin und einem Tris(hydraxymethyl)aminomethan-EDTA-Sekundär-Puffer, wobei besagter Wirkstoff auf der Basis von Chlorhexidin eine Chlorhexidin-Diglukonat-Lösung C₃₄H₅₄C₁₂N₁₀O₁₄, oder eine Chlorhexidin-Dichlorhydrat-Lösung C₂₂H₃₈C₁₄N₁₀, oder Chlorhexidin-Diacetat-Lösung C₂₆H₃₈C₁₂N₁₀O₂ ist und wobei der EDTA-Sekundär-Puffer eine dehydrierte Dinatrium-EDTA Na₂H₂EDTA.2H₂O oder eine Äthylendiamintetraessigsäure H₄EDTA ist.

2. Mittel nach Anspruch 1, das ferner einen ansäuernden oder alkalisierenden Wirkstoff beinhaltet, so dass der pH-Wert des besagten Mittels 8 ist.

3. Mittel nach Anspruch 2, wobei besagter ansäuernder Wirkstoff eine Milchsäure ist.

4. Mittel nach einem jeden der Ansprüche von 1 bis 3, wobei 100 g des besagten Mittels Folgendes beinhalten:
- 1 bis 20 g Propylenglykol;
- 0,00001 bis 0,8 g 20%-ige Chiorhexidin-Digtukonat-Lösung;
- 0,001 to 0,12 g Dinatrium-EDTA;
- 0,001 to 0,60 g Tris(hydroxymethyl)aminomethan;
- 80%-ige Milchsäurelösung, um den pH-Wert 8 zu erreichen;
- entmineralisiertes Wasser, q.s. bis 100 g.

5. Mittel nach Anspruch 4, wobei 100 g des besagten Mittels Folgendes beinhalten:
- 5 bis15 g Propylenglykol;
- 0,1-0,8 g 20%-ige Chlorhexidin-Diglukonat-Lösung;
- 0,001 - 0,12 g Dinatrium-EDTA;
- 0,001-0,60 g Tris(hydroxymethyl)aminomethan;
- 80%-ige Milchsäurelösung, um den pH-Wert 8 zu erreichen;
- entmineralisiertes Wasser, q.s. bis 100 g.

6. Mittel nach Anspruch 4, wobei 100 g des besagten Mittels Folgendes beinhalten:
- 10 g Propyienglykol;
- 0,75 g 20%-ige Chlorhexidin-Diglukonat-Lösung;
- 0,001 g Dinatrium-EDTA;
- 0,0038 g Tris(hydroxymethyl)aminomethan;
- 80%-ige Milchsäurelbsung, um den pH-Wert 8 zu erreichen;
- entmineralisiertes Wasser, q.s. bis 100 g.

## Revendications

1. Composition de détergent auriculaire pour animaux et en particulier pour chiens et chats, comprenant un agent actif à base de chlorhexidine et un tampon dérivé de tris (hydroxyméthyl) aminométhane-EDTA, ledit agent actif à base de chlorhexidine étant une solution de digluconate de chlorhexidine C₃₄H₅₄C₁₂N₁₀O₁₄, ou de dichlorhydrate de chlorhexidine C₂₂H₃₈C₁₄N₁₀ ou de diacétate de chlorhexidine C₂₆H₃₈C₁₂N₁₀O₂, ledit tampon dérivé d'EDTA étant de l'EDTA dihydrate disodique Na₂H₂EDTA.2H₂O ou de l'acide éthylène diamine tétraacétique H₄EDTA.

2. Composition selon la revendication 1, comprenant également un agent acidifiant ou alcalifiant de sorte que le pH de ladite composition soit égal à 8.

3. Composition selon la revendication 2, où ledit agent acidifiant est l'acide lactique.

4. Composition selon l'une des revendications 1 à 3, où 100 g de ladite composition comprennent :
- 1 à 20 g de propylèneglycol ;
- 0,00001 à 0,8 g de solution de digluconate de chlorhexidine à 20%;
- 0,001 à 0,12 g d'EDTA disadique ;
- 0,001 à 0,60 g de tris (hydroxyméthyl) aminométhane ;
- solution d'acide lactique à 80 %, pour atteindre un pH de 8 ;
- eau déminéralisée q. s. jusqu'à 100 g.

5. Composition selon la revendication 4, où 100 g de ladite composition comprennent :
- 5 à 15 g de propylèneglycol ;
- 0,1 à 0,8 g de solution de digluconate de chlorhexidine à 20%;
- 0,001 à 0,12 g d'EDTA disodique ;
- 0,001 à 0,60 g de tris (hydroxyméthyl) aminométhane ;
- solution d'acide lactique à 80 %, pour atteindre un pH de 8 ;
- eau déminéralisée q. s. jusqu'à 100 g.

6. Composition selon la revendication 4, où 100 g de ladite composition comprennent :
- 10 g de propylèneglycol ;
- 0,75 g de solution de digluconate de chlorhexidine à 20 % ;
- 0,001 g d'EDTA disodique ;
- 0,0038 g de tris (hydroxyméthyl) aminométhane ;
- solution d'acide lactique à 80 %, pour atteindre un pH de 8 ;
- eau déminéralisée q. s. jusqu'à 100 g.
